Europäisches Patentamt

European Patent Office

Office européen des brevets

(11) Publication number: **0 242 727 B1**

# EUROPEAN PATENT SPECIFICATION

(45) Date of publication of patent specification: **22.07.92**     (51) Int. Cl.⁵: **G01N 33/574**, G01N 33/577, //G01N33/535

(21) Application number: **87105297.3**

(22) Date of filing: **09.04.87**

The file contains technical information submitted after the application was filed and not included in this specification

(54) **Method of assaying adenocarcinoma antigens.**

(30) Priority: **09.04.86 JP 81806/86**

(43) Date of publication of application:
**28.10.87 Bulletin 87/44**

(45) Publication of the grant of the patent:
**22.07.92 Bulletin 92/30**

(84) Designated Contracting States:
**DE FR GB**

(56) References cited:
**GB-A- 2 142 428**

(73) Proprietor: **KYOWA HAKKO KOGYO CO., LTD.**
**6-1, Ohtemachi 1-chome**
**Chiyoda-ku Tokyo-to(JP)**

(72) Inventor: **Yoshida, Hajime**
**9-18, Isobe**
**Sagamihara-shi Kanagawa(JP)**
Inventor: **Hanai, Nobuo**
**9056 Eastshorewood Drive, Apt. 246**
**Mercer Island, WA 98040(US)**
Inventor: **Shitara, Kenya**
**4-17-9 Morino**
**Machida-shi Tokyo(JP)**
Inventor: **Kumagai, Akiko**
**7-4-2, Tamagawagakuen**
**Machida-shi Tokyo(JP)**

(74) Representative: **Kinzebach, Werner, Dr.**
**Patentanwälte Reitstötter, Kinzebach und**
**Partner Sternwartstrasse 4 Postfach 86 06 49**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

BACKGROUND OF THE INVENTION

Field of the Invention

This invention relates to an improvement in the field of clinical diagnosis and provides a method of assaying adenocarcinoma antigens which is applicable to clinical diagnosis of human adenocarcinomas.

Description of the Prior Art

The anti-human lung adenocarcinoma monoclonal antibody ALC-186 (hereinafter referred to as "ALC-186" for short) is a monoclonal antibody prepared by the present inventors and a serodiagnostic system using the same can detect lung adenocarcinoma at a positive rate of 48% (European Patent Application No. 86 114 025.9 filed on October 9, 1986).

The anti-human stomach adenocarcinoma monoclonal antibody AMC-382 (hereinafter referred to as "AMC-382" for short) is a monoclonal antibody prepared by the present inventors and a serodiagnostic system using the same can detect stomach cancer at a positive rate of 61.5% (European Patent Application No. 87 103 140.7 filed on March 5, 1987). GB-A-2 142 428 teaches a method for use in diagnosing human adenocarcinoma. Polyclonal or monoclonal anti-K- and anti-$P^k$ antibodies are employed to detect either the antigenic determinant K which is specific to adenocarcinomas or the antigenic determinant $P^k$ associated with determinant K. The determinants K and $P^k$ are regarded as part of the tumor marker antigen CEA (carcinoembryonic antigen).

Under the present conditions, the above-mentioned serodiagnostic system for lung adenocarcinoma which uses ALC-186 and that for stomach adenocarcinoma which uses AMC-382 are of great clinical value. In cancer detection using sera, however, an improved detection method which could possibly give higher positive rates is desired yet prior to the present invention has not been realized.

SUMMARY OF THE INVENTION

The present inventors have found that when an enzyme immuno-assay in serodiagnosis of adenocarcinomas is performed by the sandwich technique using as a first antibody a mixture of anti-human lung adenocarcinoma monoclonal antibody ALC-186 and anti-human stomach adenocarcinoma antibody AMC-382 and, as a second antibody, a mixture of ALC-186 and AMC-382 each in an enzyme-labeled form, significantly increased positive rates can be obtained as compared with the systems in which either of the monoclonal antibodies is used by itself. The present invention has been completed based on the above finding. Serodiagnostic test kits are also described.

DETAILED DESCRIPTION OF THE INVENTION

In accordance with the present invention, adenocarcinoma antigens in serum samples can be assayed by immobilizing, on a solid phase, a mixture of the anti-human lung adenocarcinoma monoclonal antibody ALC-186 and the anti-human stomach adenocarcinoma monoclonal antibody AMC-382, which serves as a first antibody, allowing a human serum sample to react with the first antibody to bind adenocarcinoma antigens, then allowing a mixture of ALC-186 and AMC-382 each in a labeled form, which serves as a second antibody, to react with the adenocarcinoma antigens bound to the first antibody and determining the quantity of the thus-bound second antibody.

According to one preferred embodiment of the invention the second antibody mixture is labeled with an enzyme selected from peroxidase, urease, alkaline phosphatase and beta-galactosidase and the quantity of a bound enzyme-labeled second antibody mixture is measured using an enzyme immunoassay method.

According to another preferred embodiment of the invention the second antibody mixture is labeled with a conventional fluorescent label and the quantity of a bound fluorescence-labeled second antibody is measured using a conventional fluorescent antibody method.

According to another preferred embodiment of the invention the second antibody mixture is labeled with a conventional immunohistological marker and the quantity of a bound second antibody which is labeled with an immunohistological marker is measured using a conventional immunohistological evaluation method.

According to yet another preferred embodiment of the invention the second antibody mixture is labeled with biotin and the quantity of a bound biotinylated second antibody is measured using a biotin-avidin binding assay.

The adenocarcinoma antigens to be detected according to the present invention preferably arise from the serum of a patient having an adenocarcinoma selected from lung adenocarcinoma, stomach adenocarcinoma and breast cancer.

According to another embodiment of the present invention a serodiagnostic test kit is provided, comprising a first container having a mixture of anti-human lung adenocarcinoma monoclonal antibody ALC-186 and anti-human stomach adenocarcinoma monoclonal antibody AMC-382, in a pharmaceutically acceptable buffer as a first antibody mixture therein, and a second container having a mixture of a labeled anti-human lung adenocarcinoma monoclonal antibody ALC-186 and a labeled anti-human stomach adenocarcinoma monoclonal antibody AMC-382, as a second antibody therein, whereby the first and the second containers are packaged together as a test kit. Preferably the second antibody in the second container is labeled with an enzyme selected from peroxidase, urease, alkaline phosphatase and beta-galactosidase.

ALC-186 and AMC-382 are monoclonal antibodies previously disclosed by the present inventors in European Patent Applications No. EP-A-218 257 and No. EP-A-235 817 filed on October 9, 1986 and March 5, 1987.

Hybridoma strains capable of producing said monoclonal antibodies, namely the strains ALC-186 and AMC-382 as so referred to herein, can be produced in the manner described below. Both strains have been deposited with the European Collection of Animal Cell Culture in Great Britain under the deposit numbers ECACC 85082903 and ECACC 86022701, respectively, under the Budapest Treaty.

Eight- to ten-week old female BALB/c or nude mice treated with pristane® [intraperitoneally administered with 0.5 ml of 2,6,10,14-tetramethylpentadecane (pristane®) and fed for 1- to 2-weeks] are intraperitoneally injected with the cells of the strain ALC-186 or AMC-382 at a dose of $2-4 \times 10^{5-7}$ cells per animal. In 10 to 21 days, the hybridoma cells produce ascites carcinoma in the mice. The ascitic fluid is collected from such a mouse, subjected to salting out with 5% ammonium sulfate, dialyzed against a solution of 1.83 g/liter of disodium phosphate, 0.21 g/liter of monopotassium phosphate and 7.65 g/liter of sodium chloride (pH 7.2; hereinafter referred to as "PBS" for short) with NaCl added to 0.5 M, and applied to a Cellulofine® GCL-2000 (Seikagaku Kogyo) column (bed volume 750 ml) at a flow rate of 15 ml/hr. An IgM fraction is collected and used as a purified monoclonal antibody.

The protein quantity is estimated by the Folin method, followed by calculation based on the absorbance at 280 nm [1.0 ($OD_{280}$) is approximately equal to 1 mg of immunoglobulin M per milliliter].

The monoclonal antibodies thus obtained are evaluated for specificity characteristics based on the reactivities with normal and tumor tissues and membrane components thereof as derived from a variety of human organs obtained from a plurality of subjects, the reactivities with a variety of normal human or tumor cell lines and membrane components derived therefrom, the reactivities with the hitherto known carcinogenic embryonic antigens (e.g., CEA) and the reactivities with normal human-derived and patient-derived sera, among others, as determined by the enzyme immunoassay method, fluorescent antibody method, immunohistological evaluation method (PAP method), etc.

Enzyme labeling of the monoclonal antibodies ALC-186 and AMC-382 is performed by the periodic acid crosslinking method [Journal of Histochemistry and Cytochemistry, 22, 1084-1091 (1971)] using, as the enzyme, peroxidase, urease, alkaline phosphatase or $\beta$-galactosidase, for instance.

For example, the periodic acid crosslinking using peroxidase may be conducted in the following manner:

Peroxidase is dissolved in 1 ml of 0.3 M sodium bicarbonate buffer (pH 8.1), 0.1 ml of a 1% ethanolic solution of fluoro-2,4-dinitrobenzene (DNP) is added, 1 ml of 0.06 M periodic acid is then added and the reaction is allowed to proceed at room temperature for 30 minutes. The reaction mixture is dialyzed against 0.01 M carbonate buffer (pH 9.5) to give a dialyzate containing peroxidase having a DNP-modified aldehyde group. To the dialyzate is added 5 mg of the antibody. After 2 hours of reaction at room temperature the reaction mixture is dialyzed against 0.01 M phosphate buffer (pH 7.2) and the dialyzate is subjected to gel filtration using a Sephadex® G-100 chromatographic column. The active fraction thus obtained is used as the enzyme labeled antibody.

When peroxidase is used as the enzyme, an ABTS substrate solution [solution of 550 mg of 2,2'-azinobis-(3-ethylbenzothiazoline-6-sulfonic acid) diammonium salt in 1 liter of 0.1 M citrate buffer (pH 4.2) with hydrogen peroxide added to 1 $\mu$l/ml just prior to use] is used and the color developed is measured in terms of the absorbance at 415 nm ($OD_{415}$ nm). For urease, a solution containing 8 mg of bromocresol purple, 100 mg of urea and 0.2 mM EDTA per 100 ml thereof and having a pH of 4.8 is used as the substrate and the measurement is performed by the method described in Journal of Immunological Methods, 53, 187-194 (1982). For alkaline phosphatase, a solution of 1 mg/ml of p-nitrophenyl phosphate in

3

0.1 M diethanolamine buffer is used as the substrate.

The enzyme labeling can also be effected through biotin-avidin bonding. Labeling with biotin can be conducted by the method described in the Journal of Histochemistry and Cytochemistry, 27, 1131-1139 (1979).

The serodiagnosis is performed as follows:

A 1:1 mixture of ALC-186 and AMC-382 each in a concentration of 10 to 100 $\mu$g/ml is used as the first antibody. The mixture is distributed into the wells of a 96-well plate for EIA (50 to 200 $\mu$l per well). The plate is allowed to stand at 4°C to room temperature for 2 hours to two-overnights. After washing with PBS, 200 $\mu$l of 1% bovine serum albumin (BSA)-PBS is added to each well. The plate is allowed to stand at 4°C to room temperature for 2 hours to overnight and then washed thoroughly with PBS. Then, 50 to 100 $\mu$l of a 1- to 100-fold dilution of a serum sample is added to each well. After allowing the wells to stand at 4°C to room temperature for 2 hours to overnight, the plate is washed thoroughly with PBS. Thereafter, a 1:1 mixture of biotinylated ALC-186 (10 to 100 $\mu$g/ml) and biotinylated AMC-382 (10 to 100 $\mu$g/ml) is added, as the second antibody, to the wells (50 to 100 $\mu$l per well) and the plate is allowed to stand at 4°C to room temperature for 2 hours to overnight. The plate is washed thoroughly with PBS, avidinbiotin-peroxidase (Vector) (10 $\mu$g/ml) is added to the wells (50 to 100 $\mu$l), the plate is allowed to stand at room temperature for 10 to 30 minutes, and then the reaction is terminated by adding a 5% SDS solution in an amount of 50 to 100 $\mu$l per well. The $OD_{415}$ value is measured for each well and the antigen quantity in the serum sample is calculated based on the intensity of the color developed. By comparing the antigen levels in the sera of healthy subjects with those in the sera of cancer patients, a normal level range is defined. When the level in question exceeds this range, the test for adenocarcinoma is regarded as positive.

The following non-limiting examples illustrate the invention in further detail. All weights and percent ages are by weight unless otherwise indicated.

## EXAMPLE 1

A 1:1 mixture of ALC-186 (10 $\mu$g/ml) and AMC-382 (10 $\mu$g/ml) was distributed as the first antibody into the wells of a 96-well plate for EIA (Flow Laboratories) (100 $\mu$l per well). After standing overnight at 4°C, the plate was washed with PBS. Then, 1% BSA-PBS was added (200 $\mu$l per well). After standing overnight at 4°C, the plate was washed thoroughly with PBS.

Ten-fold dilutions of sera of healthy subject (20 samples), sera of lung cancer patients (23 samples), sera of stomach cancer patients (20 samples), serum of a breast cancer patient (1 sample) and serum of a malignant lymphoma patient (1 sample) were respectively added to the wells in an amount of 50 $\mu$l per well. After standing overnight at 4°C, the plate was washed thoroughly with PBS. A 1:1 mixture of biotinylated ALC-186 (10 $\mu$g/ml) and biotinylated AMC-382 (10 $\mu$g/ml) was distributed, as the second antibody, into the wells (100 $\mu$l per well). After standing overnight at 4°C, the plate was washed thoroughly with PBS. Then, avidin-biotin-peroxidase (10 $\mu$g/ml) was added to the wells (100 $\mu$l per well). After standing at room temperature for 1 hour, the plate was washed thoroughly with PBS. The ABTS substrate solution mentioned hereinabove was distributed in 100 $\mu$l portions into the wells. After 30 minutes of reaction at room temperature, the reaction was terminated by adding a 5% SDS solution (100 $\mu$l per well). The color developed was measured for each well using an absorptiometer ($OD_{415}$). The results thus obtained are shown in Table 1. In control runs, serodiagnostic assaying was performed with the same serum samples in the same manner as above except that ALC-186 and AMC-382 was used by itself as the first antibody and biotinylated ALC-186 or biotinylated AMC-382 was used alone as the second antibody. The results obtained in these control runs are also shown in Table 1. The cut-off value employed for each assay system was equal to mean $OD_{415}$ + 25D (SD being standard deviation) for 20 healthy subjects used in each assay system.

As is evident from Table 1, the system using the 1:1 mixture of ALC-186 and AMC-382 gave significantly increased positive rates in cancer patients, particularly in patients with adenocarcinomas such as lung adenocarcinoma, stomach adenocarcinoma and breast cancer.

EP 0 242 727 B1

Table 1

| Serum sample | Positive rate (%)<br>(number of positive samples/total number of samples) | | |
|---|---|---|---|
| First antibody | ALC-186 | AMC-382 | ALC-186+AMC-382 |
| Second antibody | Biotinylated<br>ALC-186 | Biotinylated<br>AMC-382 | Biotinylated<br>ALC-186+<br>Biotinylated<br>AMC-382 |
| Healthy subjects | 10.0 (2/20) | 0.0 (0/20) | 0.0 (0/20) |
| Cancer patients, total | 22.2 (10/45) | 33.3 (15/45) | 53.3 (24/45) |
| Lung cancer patients, total | 26.1 (6/23) | 13.0 (3/23) | 39.1 (9/23) |
|   Lung adenocarcinoma patients | 62.5 (5/8) | 12.5 (1/8) | 87.5 (7/8) |
|   Lung squamous cell carcinoma patients | 0.0 (0/12) | 0.0 (0/12) | 0.0 (0/12) |
|   Tissue type unknown | 33.3 (1/3) | 66.7 (2/3) | 66.7 (2/3) |
| Stomach adenocarcinoma patients | 15.0 (3/20) | 60.0 (12/20) | 70.0 (14/20) |
| Breast cancer patients | 100.0 (1/1) | 0.0 (0/1) | 100.0 (1/1) |
| Malignant lymphoma patients | 0.0 (0/1) | 0.0 (0/1) | 0.0 (0/1) |

Pristane®-treated 8-week-old female BALB/c mice were intraperitoneally injected with the hybridoma strain ALC-186 in a dose of $4 \times 10^6$ cells per animal.

In 10 to 21 days, the hybridoma produced ascitic carcinoma. The ascitic fluid was collected from ascitic fluid-bearing mice (5 to 10 ml per animal), deprived of solid matter by centrifugation (3,000 rpm, 5 minutes), subjected to salting out with 50% ammonium sulfate and dialyzed against PBS supplemented with 0.5 M NaCl. The dialyzate was applied to a Cellulofine® GCL-2000 (Seikagaku Kogyo) column (bed volume 750 ml) at a flow rate of 15 ml/hr. An IgM fraction was collected and used as the purified monoclonal antibody.

REFERENCE EXAMPLE 2

Purification of monoclonal antibody AMC-382

Pristane®-treated 8-week-old female C57BL/6 mice were intraperitoneally injected with the hybridoma strain AMC-382 in a dose of $4 \times 10^6$ cells per animal.

In 10 to 21 days, the hybridoma produced ascitic carcinoma. The ascitic fluid was collected from ascitic fluid-bearing mice (5 to 10 ml per animal), deprived of solid matter by centrifugation (3,000 rpm, 5 minutes), subjected to salting out with 50% ammonium sulfate and dialyzed against PBS supplemented with 0.5 M NaCl. The dialyzate was passed through a Cellulofine® GCL-2000 (Seikagaku Kogyo) column (bed volume 750 ml) at a flow rate of 15 ml/hr. An IgM fraction was collected and used as purified antibody.

The present invention thus makes it possible to perform serodiagnosis of human adenocarcinomas in a simple, efficient and reliable manner.

**Claims**

1. A serodiagnostic method for assaying adenocarcinoma antigens in a serum sample which comprises the steps of:

   (1) immobilizing, on a solid base, a first antibody which is a mixture of anti-human lung adenocarcinoma monoclonal antibody ALC-186 obtainable from a hybridoma strain deposited under the number ECACC 85082903 and anti-human stomach adenocarcinoma monoclonal antibody AMC-382 obtainable from a hybridoma strain deposited under the number ECACC 86022701;

   (2) allowing a human serum sample to react with said first antibody to bind adenocarcinoma antigens;

   (3) allowing a second antibody, which is a mixture of anti-human lung adenocarcinoma monoclonal antibody ALC-186 and anti-human stomach adenocarcinoma monoclonal antibody AMC-382, each antibody in a labeled form, to react with said adenocarcinoma antigens; and

   (4) determining the quantity of the thus-bound second antibody.

2. The serodiagnostic method of claim 1 in which the second antibody of step (3) is labeled with an enzyme selected from peroxidase, urease, alkaline phosphatase and beta-galactosidase.

3. The serodiagnostic method of claim 1 or 2 in which the quantity of a bound enzyme-labeled second antibody is measured using an enzyme immunoassay method.

4. The serodiagnostic method of claim 1 in which the quantity of a bound fluorescence-labeled second antibody is measured using a fluorescent antibody method.

5. The serodiagnostic method of claim 1 in which the quantity of a bound second antibody which is labeled with an immunohistological marker is measured using an immunohistological evaluation method.

6. The serodiagnostic method of claim 1 in which the quantity of a bound biotinylated second antibody is measured using a biotinavidin binding assay.

7. The serodiagnostic method of claim 1 in which the adenocarcinoma antigens to be detected arise from the serum of a patient having an adenocarcinoma selected from lung adenocarcinoma, stomach adenocarcinoma and breast cancer.

8. A serodiagnostic test kit comprising:

(a) a first container having a mixture of anti-human lung adenocarcinoma monoclonal antibody ALC-186 and anti-human stomach adenocarcinoma monoclonal antibody AMC-382, in a pharmaceutically acceptable buffer as a first antibody mixture therein;

(b) a second container having a mixture of a labeled anti-human lung adenocarcinoma monoclonal antibody ALC-186 and a labeled anti-human stomach adenocarcinoma monoclonal antibody AMG-382, as a second antibody therein;

the first and the second containers packaged together as a test kit.

9.  The serodiagnostic test kit of claim 8 in which the second antibody in the second container is labeled with an enzyme selected from peroxidase, urease, alkaline phosphatase and beta-galactosidase.

**Revendications**

1.  Procédé de sérodiagnostic destiné au dosage d'antigènes d'adénocarcinome dans un échantillon de sérum, comprenant les étapes de :

(1) immobilisation, sur une base solide, d'un premier anticorps qui est un mélange d'un anticorps monoclonal anti-adénocarcinome pulmonaire humain ALC-186, obtenu à partir d'une souche d'hybridome déposée sous le numéro ECACC 85082903, et d'un anticorps monoclonal anti-adénocarcinome gastrique humain AMC-382, obtenu à partir d'une souche d'hybridome déposée sous le numéro ECACC 86022701;

(2) mise à réagir d'un échantillon de sérum humain avec ledit premier anticorps pour fixer des antigènes d'adénocarcinome ;

(3) mise à réagir d'un second anticorps qui est un mélange d'un anticorps monoclonal anti-adénocarcinome pulmonaire humain ALC-186 et d'un anticorps monoclonal anti-adénocarcinome gastrique humain AMC-382, chaque anticorps se présentant sous une forme marquée, avec lesdits antigènes d'adénocarcinome ; et

(4) détermination de la quantité du second anticorps ainsi fixé.

2.  Procédé de sérodiagnostic selon la revendication 1, dans lequel le second anticorps de l'étape (3) est marqué par une enzyme sélectionnée parmi la peroxydase, l'uréase, la phosphatase alcaline et la $\beta$-galactosidase.

3.  Procédé de sérodiagnostic selon la revendication 1 ou 2, dans lequel la quantité d'un second anticorps fixé, marqué par une enzyme, est mesurée à l'aide d'un procédé de dosage immuno-enzymatique.

4.  Procédé de sérodiagnostic selon la revendication 1, dans lequel la quantité d'un second anticorps fixé, marqué par fluorescence, est mesurée à l'aide d'un procédé à anticorps fluorescents.

5.  Procédé de sérodiagnostic selon la revendication 1, dans lequel la quantité d'un second anticorps fixé, marqué par un marqueur immuno-histologique, est mesurée à l'aide d'un procédé d'évaluation immuno-histologique.

6.  Procédé de sérodiagnostic selon la revendication 1, dans lequel la quantité d'un second anticorps fixé biotinylé est mesurée à l'aide d'un procédé de fixation de biotine-avidine.

7.  Procédé de sérodiagnostic selon la revendication 1, dans lequel les antigènes d'adénocarcinome à détecter proviennent du sérum d'un malade porteur d'un adénocarcinome sélectionné parmi l'adénocarcinome pulmonaire, l'adénocarcinome gastrique et le cancer du sein.

8.  Trousse de sérodiagnostic comprenant:

(a) un premier conteneur contenant un mélange d'un anticorps monoclonal anti-adénocarcinome pulmonaire humain ALC-186 et d'un anticorps monoclonal anti-adénocarcinome gastrique humain AMC-382, dans un tampon pharmaceutiquement acceptable, comme premier mélange d'anticorps ;

(b) un second conteneur contenant un mélange d'un anticorps monoclonal anti-adénocarcinome pulmonaire humain ALC-186 et d'un anticorps monoclonal anti-adénocarcinome gastrique humain AMC-382, comme second anticorps ; le premier et le second conteneur étant sous la forme d'une trousse de diagnostic.

9. Trousse de sérodiagnostic selon la revendication 8, dans laquelle le second anticorps présent dans le second conteneur est marqué par une enzyme sélectionnée parmi la peroxydase, l'uréase, la phosphatase alcaline et la *β*-galactosidase.

**Patentansprüche**

1. Serodiagnostisches Verfahren zur Bestimmung von Adenocarcinom-Antigenen in einer Serumprobe, wobei man

   (1) einen ersten Antikörper an einer festen Basis immobilisiert, der eine Mischung ist

   aus dem monoklonalen anti-Human-Lungenadenocarcinom-Antikörper ALC-186, der erhältlich ist aus einem unter der Nummer ECACC 85082903 hinterlegten Hybridom-Stamm, und

   aus dem monoklonalen anti-Human-Magenadenocarcinom-Antikörper AMC-382, der erhältlich ist aus einem unter der Nummer ECACC 86022701 hinterlegten Hybridom-Stamm;

   (2) eine Humanserumprobe mit dem ersten Antikörper reagieren läßt, um die Adenocarcinom-Antigene zu binden;

   (3) einen zweiten Antikörper,

   der eine Mischung

   aus dem monklonalen anti-Human-Lungenadenocarcinom-Antikörper ALC-186 und

   aus dem monklonalen anti-Human-Magenadenocarcinom-Antikörper AMC-382 ist,

   mit den Adenocarcinom-Antigenen reagieren läßt; und

   (4) die Menge des so gebundenen zweiten Antikörper bestimmt.

2. Serodiagnostisches Verfahren nach Anspruch 1, worin der zweite Antikörper der Stufe (3) mit einem Enzym markiert wird, das ausgewählt wird unter Peroxidase, Urease, alkalischer Phosphatase und beta-Galactosidase.

3. Serodiagnostisches Verfahren nach Anspruch 1 oder 2, worin die Menge eines gebundenen Enzym-markierten zweiten Antikörpers unter Anwendung eines Enzym-Immunoassays bestimmt wird.

4. Serodiagnostisches Verfahren nach Anspruch 1, worin die Menge eines gebundenen fluoreszenzmarkierten zweiten Antikörpers unter Anwendung einer Fluoreszenz-Antikörper-Methode bestimmt wird.

5. Serodiagnostisches Verfahren nach Anspruch 1, worin die Menge eines gebundenen zweiten, mit einem immunohistologischen Marker markierten Antikörpers unter Anwendung einer immunohistologischen Auswertungsmethode bestimmt wird.

6. Serodiagnostisches Verfahren nach Anspruch 1, worin die Menge eines gebundenen biotinylierten zweiten Antikörpers unter Anwendung eines Biotin-Avidin-Bindungsassays bestimmt wird.

7. Serodiagnostisches Verfahren nach Anspruch 1, worin die zu bestimmenden Adenocarcinom-Antigene von dem Serum eines Patienten mit einem Adenocarcinom stammen, das ausgewählt ist unter Lungen-Adenocarcinom, Magen-Adenocarcinom und Brustkrebs.

8. Serodiagnostischer Testkit umfassend:

   (a) einen ersten Behälter mit darin enthaltener Mischung aus monoklonalen anti-Human-Lungencarcinom-Antikörpern ALC-186 und aus monoklonalen anti-Human-Magenadenocarcinom-Antikörpern AMC-382 in einem pharmazeutisch verträglichen Puffer als erste Antikörpermischung;

   (b) einem zweiten Behälter mit darin enthaltener Mischung aus markierten monoklonalen anti-Human-Lungencarcinom-Antikörpern ALC-186 und aus markierten monoklonalen anti-Human-Magenadenocarcinom-Antikörpern AMC-382 als zweite Antikörper;

   wobei der erste und der zweite Behälter zusammen als Testkit verpackt sind.

9. Serodiagnostischer Testkit nach Anspruch 8, worin der zweite Antikörper in dem zweiten Behälter mit einem Enzym markiert ist, das ausgewählt ist unter Peroxidase, Urease, alkalischer Phosphatase und beta-Galactosidase.